# EUROPÄISCHE PATENTSCHRIFT

(11) **EP 2 191 862 B1**
(45) Veröffentlichungstag und Bekanntmachung des Hinweises auf die Patenterteilung: **15.07.2015**
(21) Anmeldenummer: 09175460.6
(22) Anmeldetag: 10.11.2009
(51) Int. Cl.: A61N 1/375, A61N 1/372, A61N 1/05

(54) **Adapter, Adapter-Verkaufseinheit und System aus dem Adapter, einem implantierbaren medizinelektronischen Gerät und einer Elektrodenleitung**
Adapter, adapter sales unit and system comprising the adapter, implantable medical-electronic device and electrode wire
Adaptateur, unité de vente d'adaptateur et système constitué de l'adaptateur, d'un appareil médical électronique implantable et d'une ligne d'électrodes

(30) Priorität: 01.12.2008 DE 102008044223
(43) Veröffentlichungstag der Anmeldung: 02.06.2010
(73) Patentinhaber: Biotronik CRM Patent AG, 6341 Baar (CH)
(72) Erfinder: Dörr, Thomas, 12437, Berlin (DE)
(74) Vertreter: Lindner-Vogt, Karin L.

(56) Entgegenhaltungen:
- EP-A1- 0 400 592
- EP-A1- 1 644 080
- US-A- 5 919 213
- US-A1- 2002 143 376
- US-A1- 2007 099 487

## Beschreibung

Die Erfindung betrifft einen Adapter zur elektrischen Verbindung eines implantierbaren medizinelektronischen Geräts mit einer Elektrodenleitung. Sie betrifft des Weiteren eine Verkaufseinheit eines solchen Adapters sowie ein System aus dem Adapter, dem implantierbaren medizinelektronischen Gerät und einer Elektrodenleitung.

Implantierbare medizinelektronische Geräte zur Unterstützung bestimmter Körperfunktionen, wie etwa Herzschrittmacher und implantierbare Defibrillatoren zur Unterstützung der Herzfunktion in pathologischen Herzzuständen, sind seit langem bekannt und im klinischen Einsatz. An diese Geräte ist im implantierten Zustand (und somit im Gebrauchszustand), eine Elektrodenleitung dauerhaft angeschlossen, um elektrische Stimulations- oder Schockimpulse, die von entsprechenden Einrichtungen im Gerät erzeugt werden, dem reizbaren Herzgewebe zuzuführen, das sich entfernt vom Gerät befindet.

Im Verlaufe der Implantation eines solchen Gerätes und der dazugehörigen Elektrodenleitung ist es erforderlich, am jeweiligen Ort der Platzierung der Elektrodenleitung bestimmte elektrische Parameter derselben und/oder Körperfunktionsparameter des Patienten unter Nutzung der Elektrodenleitung zu erfassen. Falls sich dabei Werte ergeben, die dauerhaft einwandfreie Funktionen der Gesamtanordnung nicht zuverlässig erwarten lassen, ist eine Repositionierung der Elektrodenleitung zu einem geeigneteren Wirkungsort vorzusehen, und im Zuge einer solchen Repositionierung sind mehrfache Messungen der besagten Parameter erforderlich. Üblich ist es, während des Implantationsvorgangs die Elektrodenleitung zu diesem Zweck über Signalleitungen mit den erforderlichen Messgeräten zu verbinden.

Relevanter Stand der Technik ist durch EP0400592A1, EP1644080A1 und US5919213 offenbart.

Mit Einführung der weitreichenden RF-Telemetrie zur Kommunikation zwischen einem implantierbaren medizinelektronischen Gerät (nachfolgend auch bezeichnet als "elektronisches Implantat") und dem Programmier- und Testgerät wird es möglich, während einer Implantation gänzlich auf Kabel zwischen dem Patienten und dem Programmier- und Testgerät zu verzichten, wenn während der Implantation die Messung der Elektrodenparameter (wie z.B. bei einer ICD-Implantation: Elektrodenimpedanz, Reizschwelle, P- und R-Wellen-Amplituden) vollständig vom elektronischen Implantat unterstützt werden. In diesen Fällen wäre es möglich, schon mit derzeit verfügbaren Implantaten alle Parameter zu messen und kabellos an das Programmier- und Testgerät einschließlich der benötigten Elektrogramme zu übertragen.

Nachteil dieser Lösung ist jedoch die z. T. kritische und mehrfach nötige Kontaktierung zwischen den Elektrodensteckern und dem elektronischen Implantat, da bei der Implantation die Elektroden in ihrer Implantationsreihenfolge einzeln gemessen werden. Außerdem werden, wie bereits erwähnt, die Elektroden zwischen den Messungen bei Bedarf repositioniert. Für die Kontaktierung zwischen Elektroden und elektronischen Implantat sind aufgrund der Anforderungen an eine zuverlässige permanente Kontaktierung im implantierten Zustand relativ große Ein- und Auszugskräfte und der Einsatz eines Drehmomentenschraubendrehers nötig.

Im Vergleich zur herkömmlichen Elektrodenmessung, die mittels Kabel durchgeführt wird und eine sehr einfache temporären Kontaktierung der Elektroden gestattet (durch Kroko-Klemmen oder Hülsen mit Federkontakten), ist der Aufwand einer kabellosen implantatbasierten Messung mit mehrfachem Einstecken der Elektroden in den Header des elektronischen Implantates ungerechtfertigt hoch.

Die Aufgabe der Erfindung besteht daher darin, ein elektronisches Implantat während der intraoperativen Messung temporär und effizient mit den zu implantierenden Elektroden für die intraoperative Messung zu verbinden, insbesondere ohne dass ein Werkzeug benötigt wird. Das Verfahren muss gegenüber der derzeit genutzten kabelgebundenen Messung vergleichbar einfach sein, jedoch den Vorteil bieten, gänzlich auf Kabel zwischen sterilem OP-Bereich und dem Programmier- und Testgerät verzichten zu können.

Diese Aufgabe wird gelöst durch einen Adapter mit den Merkmalen des Anspruchs 1. Zweckmäßige Fortbildungen des Erfindungsgedankens sind Gegenstand der hiervon abhängigen Ansprüche. Weiterhin wird die Aufgabe, gemäß einem relativ unabhängigen Aspekt der Erfindung, gelöst durch eine Adapter-Verkaufseinheit nach Anspruch 4 sowie, nach einem weiteren relativ unabhängigen Aspekt der Erfindung, durch ein System gemäß Anspruch 9.

Die erfindungsgemäße Lösung bietet den Vorteil, dass in Kombination mit einer weitreichenden RF-Telemetrie und den im elektronischen Implantat implementierten Messfunktionen eine intraoperative Testung ganz ohne Kabel zwischen Programmier- und Testgerät und dem sterilen Feld im OP möglich wird. Die Kontaktierung der Elektroden wird dabei ebenso einfach und effizient wie in einer kabelgebundenen Lösung. Eine Beschädigung der Elektroden durch mehrfaches Ein- und Ausstecken wird vermieden. Die Adapterlösung ist zudem preiswerter in der Herstellung zu realisieren. Ebenso können mit diesem Verfahren Verfälschungen der Messergebnisse durch lange Kabel vermieden werden.

Ein weiterer Vorteil besteht darin, dass der RF-Programmer nun keine galvanische Verbindung mit dem Patienten aufweisen muss und daher hinsichtlich der Patientenableit- und -fehlerströme in eine andere Schutzklasse fällt. Die senkt die Kosten für ein derartiges Programmiergerät zusätzlich, neben der Kosteneinsparung für das nun nicht mehr benötigte "Implantationsmodul".

Das vorgeschlagene System hat in einer bevorzugten Ausführung folgende Merkmale:
(a) Es umfasst ein elektronisches Implantat, das zu seinem funktionsgemäßen Einsatz mit mindestens einer zu implantierenden Elektrode zu verbinden ist, wobei es gebräuchlich ist, im Rahmen der Implantation mindestens einen (elektrischen) Parameter der Elektrode zu bestimmen, um die korrekte Platzierung der Elektrode zu verifizieren.
(b) Das elektronische Implantat verfügt über eine "weitreichende Telemetrie", die die Übertragung von Messwerten über eine Distanz von >2m erlaubt, und ist in der Lage, mindestens einen für die Elektrodenplatzierung relevanten Parameter zu messen.
(c) Das System verfügt über einen zusätzlichen sterilen Adapter zur temporären Kontaktierung zwischen elektronischen Implantat und Elektrode(n).
(d) Der zusätzliche Adapter ist derart ausgeführt, dass zur Kontaktierung und Dekontaktierung zwischen elektronischem Implantat und Elektrode kein Werkzeug benötigt wird.

Gemäß einzelnen sinnvollen Ausgestaltungen des Erfindungsgedankens hat die vorgeschlagene Lösung eines oder mehrere der folgenden Merkmale:
(a) Der zusätzliche Adapter wird in einer Sterilverpackung ausgeliefert.
(b) Der zusätzliche Adapter wird in der Sterilverpackung des elektronischen Implantates ausgeliefert.
(c) Der zusätzliche Adapter wird in der Sterilverpackung der Elektrode ausgeliefert.
(d) Der zusätzliche Adapter wird vormontiert (bereits in dem Header des Implantates eingesteckt) in der Sterilverpackung des elektronischen Implantates ausgeliefert.
(e) Der zusätzliche Adapter wird vormontiert in der Sterilverpackung der Elektrode ausgeliefert.
(f) Die Kontaktierung des Adapters mit dem elektronischen Implantat erfolgt über Federkontakte, wobei die folgenden Standards berücksichtigt werden: IS-1, IS-4, DF-1.
(g) Die Kontaktierung des Adapters mit der Elektrode erfolgt über Federkontakte.
(h) Die Einsteckkräfte für den Adapter in das elektronische Implantat überschreiten nicht 14 Dekanewton.
(i) Die Auszugskraft für den Adapter aus dem elektronischen Implantat überschreitet nicht 14 Dekanewton.
(j) Das Implantat ist in der Lage, alle für die jeweilige Anwendung erforderlichen Parameter zur Elektrodenplatzierung zu messen.
(k) Das Implantat verfügt über eine RF-Telemetrie.
(l) Das Implantat verfügt über eine RF-Telemetrie im MICS-Band.

Vorteil und Zweckmäßigkeiten der Erfindung ergeben sich im Übrigen aus der nachfolgenden Beschreibung von Ausführungsbeispielen anhand der Figuren. Von diesen zeigen:
- Fig. 1: eine schematische Gesamtdarstellung eines erfindungsgemäßen Systems,
- Fig. 2: eine schematische Darstellung einer Ausführungsform des erfindungsgemäßen Systems,
- Fig. 3: eine Detaildarstellung der Fixierung eines erfindungsgemäßen Adapters im Header eines das implantierbare Gerät darstellenden Herzschrittmachers und
- Fig. 4a und 4b: Ansichten eines weiteren Beispiels einer Anordnung mit einem Adapter, wobei Fig. 4a ein Elektroden Leitungsende mit aufgesetzten Adapter während der Einführung und Fig. 4b den Adapter im Gebrauchszustand zeigt.

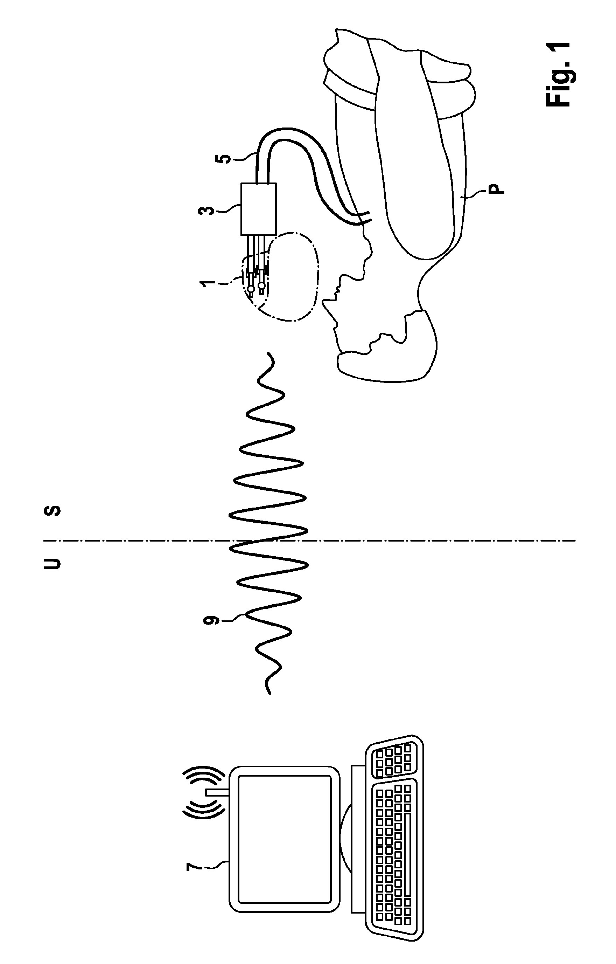

In Fig. 1 ist ein Gesamtsystem, bestehend aus einem Herzschrittmacher 1, einem Elektrodenadapter 3 zur temporären Kontaktierung einer implantierbaren Elektrodenleitung 5 und dem Programmier- und Testgerät 7 dargestellt. Das Programmier- und Testgerät befindet sich in diesem Aufbau in einem unsterilen Bereich U im Operationssaal. Die Elektroden, implantiert im Patienten P, der Herzschrittmacher 1 und der Elektrodenadapter 3 sind als sterile Komponenten im sterilen Bereich S des OP angeordnet. Mittels RF-Telemetrie 9 erfolgt die Steuerung und Datenübertragung der intraoperativen Messungen, wie z.B. P-, R-Wellenamplituden, Stimulationsreizschwellen, Elektrodenimpedanzen.

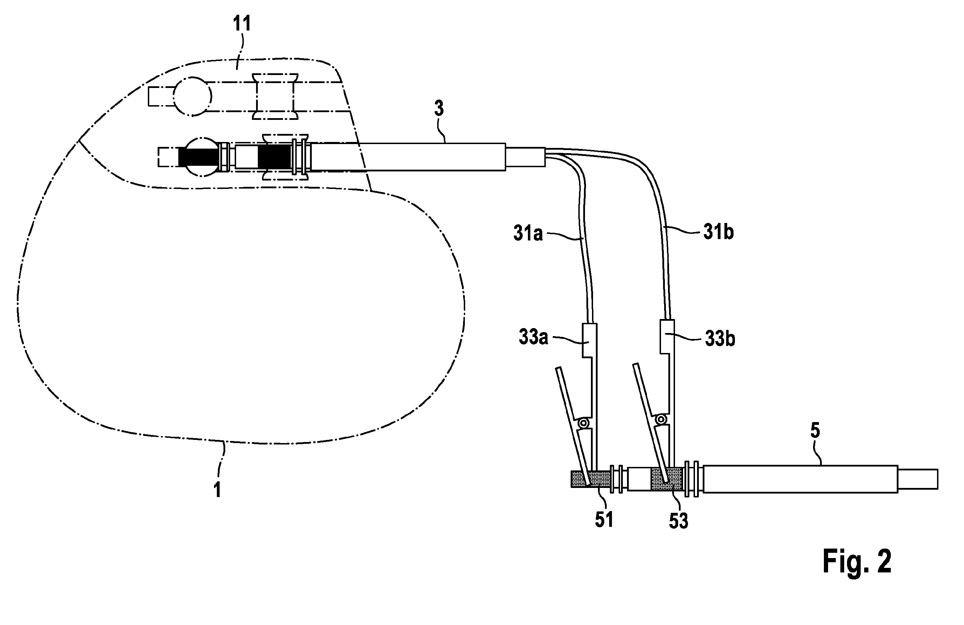

In der Fig. 2 ist eine einfache Ausführung des Adapters 3 zur temporären Elektrodenkontaktierung dargestellt. Hier wird ein IS-1-Stecker (alternativ IS-4, DF-1) bei Auslieferung des Herzschrittmachers 1 bereits im Header 11 kontaktiert. Dieser Adapter führt die elektrischen Verbindungen mittels zweier Kabel 31a, 31b zu zwei Krokodilklemmen 33a, 33b und erlaubt so die einfache Kontaktierung der zu testenden Elektrodenleitung 5, indem die Krokodilklemmen an deren Steckerkontakte 51, 53 angeklemmt werden.

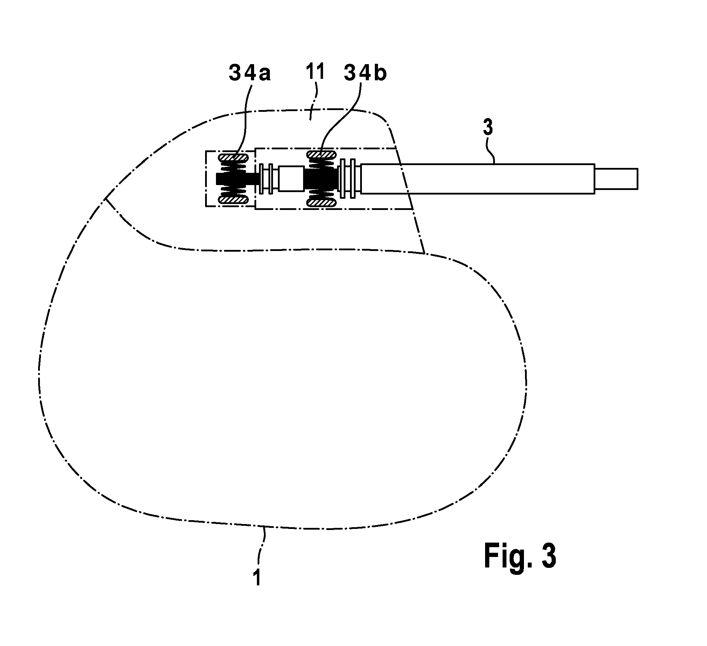

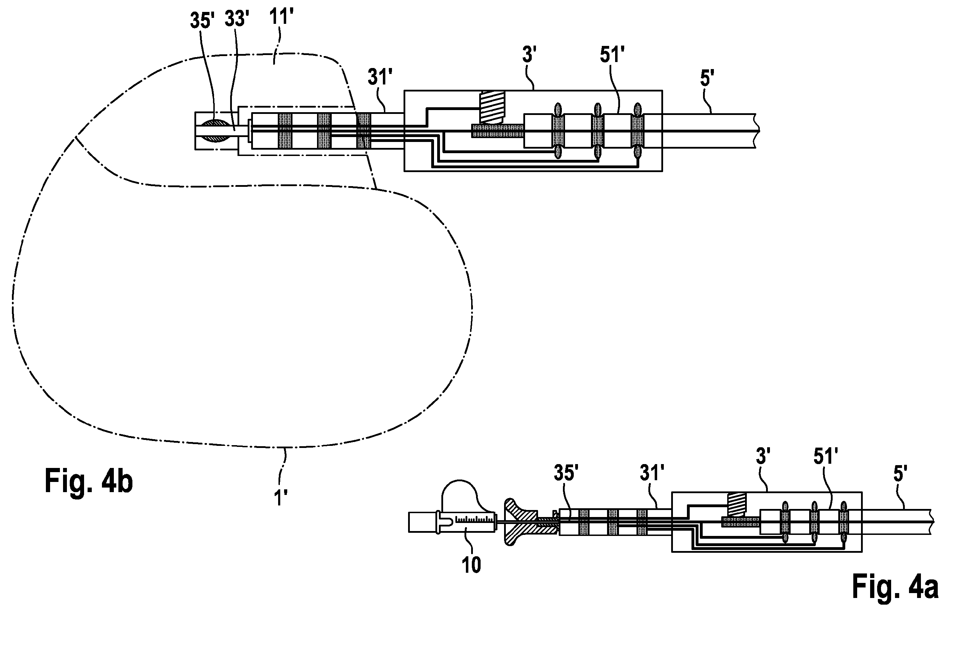

In Fig. 3 ist ein Ausführungsdetail des vormontierten Elektrodenadapters 3 dargestellt. Sie zeigt die Kontaktierung dieses Adapters mittels Federkontakten 34a, 34b im Header 11 des Herzschrittmachers 1. Mit dieser Anordnung ist es möglich, den Adapter nach Abschluss der intraoperativen Messungen ohne Werkzeug zu entfernen, indem er einfach mit mäßiger Auszugskraft aus dem Header herausgezogen wird. In Fig. 4a und 4b ist als alternative Ausführung ein Elektrodenadapter 3' dargestellt, der jedoch nicht Teil der Erfindung ist.

In dieser Ausführungsform wird der Adapter nicht zusammen mit dem elektronischen Implantat, sondern vormontiert auf dem Stecker 51' einer der zu implantierenden Elektrodenleitung 5' ausgeliefert.

In diesem Beispiel ist eine 4-polare Elektrode dargestellt. Auf den Stecker 5' dieser Elektrodenleitung ist der Elektrodenadapter 3' entsprechend der IS-4 Norm mittels Federkontaktierung aufgesteckt, der wiederum einen IS-4-Stecker 31' umfasst, dessen Pin 33' einen Federkontakt 35' zu Kontaktierung im Header 11' des Herzschrittmachers 1' beinhaltet. Des Weiteren sind die Durchmesser des Adaptersteckers 31' an der unteren Toleranzgrenze oder geringfügig unter den im IS-4 Standard definierten Maßen ausgeführt, so dass die Ein- und Aussteckkräfte gegenüber der Elektrode geringer ausfallen. So ist eine mehrfache Kontaktierung einfach und ohne Werkzeug möglich.

Außerdem enthält dieser Adapter eine zentrale Bohrung 35' zur Einführung eines Mandrins 10 zur Implantation der Elektrode, so dass dieser Adapter zur Elektrodenimplantation nicht entfernt werden muss. Die der Elektrode zugeordneten Mandrins sind um die Adapterlänge entsprechend verlängert.

## Patentansprüche

1. Adapter(3) zur temporären, sterilen elektrischen Verbindung eines implantierbaren medizinelektronischen Geräts(1) mit einer an das Gerät anzuschließenden einen Steckerabschnitt umfassenden Elektrodenleitung(5) während der Implantation zur unverfälschten Übertragung von an der Elektrodenleitung(5) erfassbaren Messwerten in das Gerät(1), wobei der Adapter(3) ausgebildet ist mit einem gleich dem Steckerabschnitt der Elektrodenleitung(5) ausgeführten Steckerabschnitt mit mindestens einem Kontakt(51, 53) sowie einer der Anzahl der Kontakte am Steckerabschnitt entsprechenden Anzahl von Signalkabeln(31a, 31b), wobei das Gerät erste Fixierungsmittel zur lösbaren Fixierung des Adapters am Gerät aufweist, und der Adapter zweite Fixierungsmittel zur lösbaren Fixierung des Adapters(3) auf der Elektrodenleitung (5) aufweist,
**dadurch gekennzeichnet, dass** die zweiten Fixierungsmittel als Krokodilklemmen(33a, 33b), zum Fixieren des oder jedes Signalkabels(31a, 31b) auf dem oder jeweils einem Kontakt(51, 53) eines Elektrodenleitungs-Steckers ausgebildet sind.

2. Adapter(3) nach Anspruch 1, wobei der Steckerabschnitt mehrere, insbesondere als Ringkontakte gebildete Außenkontakte aufweist.

3. Adapter(3) nach einem der vorangehenden Ansprüche, ausgebildet zur werkzeuglosen Verbindung sowohl mit dem Gerät(1) als auch der Elektrodenleitung(5).

4. Adapter-Verkaufseinheit, beinhaltend den Adapter (3) nach einem der vorangehenden Ansprüche, sowie eine Sterilverpackung.

5. Adapter-Verkaufseinheit nach Anspruch 4,
wobei die Sterilverpackung zugleich eine Elektrodenleitung(5) enthält, an die der Adapter(3) angepasst ist.

6. Adapter-Verkaufseinheit nach Anspruch 5, wobei der Adapter(3) mit der Elektrodenleitung(5) vormontiert ist.

7. Adapter-Verkaufseinheit nach Anspruch 5, wobei die Sterilverpackung zugleich ein medizinelektronisches Gerät(1) enthält, an das der Adapter(3) angepasst ist.

8. Adapter-Verkaufseinheit nach Anspruch 7, wobei der Adapter(3) mit dem Gerät(1) vormontiert ist.

9. System aus einem Adapter (3) nach einem der Ansprüche 1 bis 4 und einem implantierbaren medizinelektronischen Gerät(1) zum Einsatz mit einer an das Gerät(1) anzuschließender Elektrodenleitung(5), wobei das Gerät über Messmittel verfügt zur Messung innerhalb eines sterilen Operationsbereichs von an der Elektrodenleitung(5) erfassbaren Messwerten, wie etwa einer Elektrodenimpedanz, einer Reizschwelle oder von Amplituden von Herzaktionspotenzialen, wobei das Gerät weiterhin über eine Sendeeinheit verfügt zur drahtlosen Übertragung der Messwerte nach außerhalb des sterilen Operationsbereichs.

10. System nach Anspruch 9, wobei die ersten Fixierungsmittel Federmittel (34a, 34b), insbesondere Federkontakte (34a, 34b) zur Fixierung in einem Header-Abschnitt (11) des Gerätes (1) aufweisen.

11. System nach einem der Ansprüche 9 oder 10,
wobei die Sendeeinheit eine Sendereichweite von 2 m oder mehr aufweist.

12. System nach einem der Ansprüche 9 bis 11,
wobei die Sendeeinheit als RF-Telemetrieeinheit ausgebildet ist, welche insbesondere im MICS-Band arbeitet.

13. System nach einem der Ansprüche 9 bis 12, beinhaltend eine an das Gerät anschließbare Elektrodenleitung (5).

## Claims

1. An adapter (3) for the temporary, sterile electrical connection of an implantable medical electronic device (1) comprising an electrode lead (5) having a plug section for connection to the device during implantation for the unbiased transmission of measurement values, which can be detected at the electrode lead (5), into the device (1), wherein the adapter (3) is designed with a plug section, which is identical to the plug section of the electrode lead (5) and has at least one contact (51, 53) and a number of signal cables (31a, 31b) corresponding to the number of contacts on the plug section, wherein the device comprises first fixing means for the detachable fixation of the adapter (3) on the device (1), and wherein the adapter (3) comprises second fixing means for the detachable fixation of the adapter (3) on the electrode lead (5),
**characterized in that** the second fixing means are designed as alligator clips (33a, 33b) for fixing the or each signal cable (31a, 31b) on the or each contact (51, 53) of an electrode lead plug.

2. The adapter (3) according to claim 1, wherein the plug section comprises a plurality of outer contacts, which are designed as annular contacts in particular.

3. The adapter (3) according to any one of the preceding claims, said adapter being connectable to the device (1) and to the electrode lead (5) without the use of tools.

4. An adapter sales unit containing the adapter (3) according to any one of the preceding claims, and containing sterile packaging.

5. The adapter sales unit according to claim 4, wherein the sterile packaging also contains an electrode lead (5), to which the adapter (3) is adapted.

6. The adapter sales unit according to claim 5, wherein the adapter (3) is preassembled with the electrode lead (5).

7. The adapter sales unit according to claim 5, wherein the sterile packaging also contains a medical electronic device (1), to which the adapter (3) is adapted.

8. The adapter sales unit according to claim 7, wherein the adapter (3) is preassembled with the device (1).

9. The system comprising an adapter (3) according to any one of claims 1 to 4 and comprising an implantable medical electronic device (1) for use with an electrode lead (5) for connection to the device (1), wherein the device has measurement means for performing a measurement, within a sterile operating area, of measurement values that can be detected at the electrode lead (5), such as electrode impedance, a stimulation threshold, or amplitudes of cardiac action potentials, wherein the device further comprises a transmitter unit for the wireless transmission of the measurement values to the outside of the sterile operating area.

10. The system according to claim 9, wherein the first fixing means comprise spring means (34a, 34b), in particular spring contacts (34a, 34b), for fixation in a header section (11) of the device (1).

11. The system according to any one of claims 9 or 10, wherein the transmitter unit has a transmission range of 2 m or more.

12. The system according to any one of claims 9 to 11, wherein the transmitter unit is designed as an RF telemetry unit, which functions in the MICS band in particular.

13. The system according to any one of claims 9 to 12, containing an electrode lead (5), which can be connected to the device.

## Revendications

1. Adaptateur (3) pour la liaison électrique temporaire, stérile d'un appareil (1) électronique médical implantable avec une ligne d'électrode (5) comprenant une section de raccordement à brancher sur l'appareil pendant l'implantation pour la transmission non faussée de valeurs de mesures pouvant être captées sur la ligne d'électrode (5) dans l'appareil (1), où l'adaptateur (3) est conçu avec une section de raccordement réalisée identique à la section de raccordement de la ligne d'électrode (5) avec au moins un contact (51, 53) ainsi qu'avec un nombre de câbles de signal (31a, 31b) correspondant au nombre de contacts sur la section de raccordement , où l'appareil présente des premiers moyens de fixation pour la fixation amovible de l'adaptateur sur l'appareil, et l'adaptateur présente des deuxièmes moyens de fixation pour la fixation amovible de l'adaptateur (3) sur la ligne d'électrode (5),
**caractérisé en ce que** les deuxièmes moyens de fixation sont conçus sous forme de pinces crocodiles (33a, 33b) pour la fixation du, ou de chaque, câble de signal (31a, 31b) sur le, ou un, contact (51, 53) d'une prise de la ligne d'électrode.

2. Adaptateur (3) selon la revendication 1, dans lequel la section de raccordement présente plusieurs contacts externes, notamment conçus sous forme de contacts annulaires.

3. Adaptateur (3) selon l'une des revendications précédentes, conçu pour la liaison sans outils à la fois avec l'appareil et également avec la ligne d'électrode (5).

4. Unité de vente d'adaptateur, contenant l'adaptateur (3) selon l'une des revendications précédentes, ainsi qu'un emballage stérile.

5. Unité de vente d'adaptateur selon la revendication 4, dans lequel l'emballage stérile contient simultanément une ligne d'électrode (5) à laquelle est adapté l'adaptateur (3).

6. Unité de vente d'adaptateur selon la revendication 5, dans lequel l'adaptateur (3) est monté préalablement avec la ligne d'électrode (5)

7. Unité de vente d'adaptateur selon la revendication 5, dans lequel l'emballage stérile contient simultanément un appareil (1) électronique médical auquel est adapté l'adaptateur (3).

8. Unité de vente d'adaptateur selon la revendication 7, dans lequel l'adaptateur (3) est monté préalablement avec l'appareil (1).

9. Système constitué d'un adaptateur (3) selon l'une des revendications 1 à 4, et d'un appareil (1) électronique médical implantable, pour la mise en oeuvre avec une ligne d'électrode (5) à relier avec l'appareil (1), où l'appareil dispose de moyens de mesure pour la mesure de valeurs de mesures comme, par exemple, une impédance d'électrode, un seuil d'excitabilité ou des amplitudes de potentiels d'action cardiaque, pouvant être avec saisies par la ligne d'électrode (5) dans un domaine opératoire stérile, où l'appareil dispose en outre d'une unité émettrice pour la transmission sans fil des valeurs de mesure vers l'extérieur du domaine opératoire stérile.

10. Système selon la revendication 9, dans lequel les premiers moyens de fixation présentent des moyens de type ressort (34a, 34b), notamment des contacts à ressort (34a, 34b) pour la fixation dans une section Header (11) de l'appareil (1).

11. Système selon l'une des revendications 9 ou 10, dans lequel l'unité émettrice présente une plage de transmission de 2 m ou plus.

12. Système selon l'une des revendications 9 à 11, dans lequel l'unité émettrice est conçue sous la forme d'une unité de télémétrie RF, laquelle travaille en particulier dans la bande MICS.

13. Système selon l'une des revendications 9 à 12, contenant une ligne d'électrode (5) pouvant se raccorder à l'appareil.
